(19) Europäisches Patentamt
European Patent Office
Office européen des brevets

(11) EP 4 253 394 A1

(12) **EUROPEAN PATENT APPLICATION**
published in accordance with Art. 153(4) EPC

(43) Date of publication:
**04.10.2023 Bulletin 2023/40**

(21) Application number: **21897078.8**

(22) Date of filing: **25.11.2021**

(51) International Patent Classification (IPC):
*C07H 13/08* (2006.01) *C07D 311/64* (2006.01)
*C07C 69/88* (2006.01) *A61K 31/7028* (2006.01)
*A61K 31/352* (2006.01) *A61K 31/235* (2006.01)
*A61P 31/14* (2006.01) *A61P 9/00* (2006.01)
*A61P 11/00* (2006.01) *A61P 35/00* (2006.01)
*A61P 19/02* (2006.01) *A61P 29/00* (2006.01)
*A61P 13/00* (2006.01) *A61P 1/12* (2006.01)

(86) International application number:
**PCT/CN2021/133118**

(87) International publication number:
**WO 2022/111578 (02.06.2022 Gazette 2022/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.11.2020 CN 202011341469**

(71) Applicant: **Shenzhen Cell Inspire Pharmaceutical Development Co., Ltd.**
**Shenzhen, Guangdong 518108 (CN)**

(72) Inventors:
- **XU, Jun**
  **Shenzhen, Guangdong 518108 (CN)**
- **HAO, Mengjiao**
  **Shenzhen, Guangdong 518108 (CN)**
- **ZHANG, Yuting**
  **Shenzhen, Guangdong 518108 (CN)**
- **CHEN, Hao**
  **Shenzhen, Guangdong 518108 (CN)**

(74) Representative: **DehnsGermany Partnerschaft von Patentanwälten**
**Theresienstraße 6-8**
**80333 München (DE)**

(54) **PREPARATION METHOD FOR AND APPLICATION OF CLASS OF STELLATE BIFUNCTIONAL COMPOUNDS TARGETING SPIKE PROTEIN AGAINST RESPIRATORY TRACT INFECTION VIRUS AND SALT THEREOF**

(57) Provided are stellate compounds that target spike proteins and have a significant anti-SARS-CoV-2 ability and a certain broad spectrum. Such molecules and salts thereof have at least one basic unit R(X,, which binds at an orthotopic binding site such as an MD domain or an allosteric site, to a virus containing spike proteins or a virus having spike proteins on its surface, thereby preventing coronavirus or other viruses which express spike proteins on their surfaces from invading host cells, and preventing the occurrence of viral infection. In addition, interaction of the molecules and salts thereof with vitamin K-dependent proteins in the human body inhibits the expression of vitamin K so as to inhibit blood coagulation in the human body, thereby treating thrombosis caused by coronavirus and producing a curative effect for pneumonia caused by a severe viral infection.

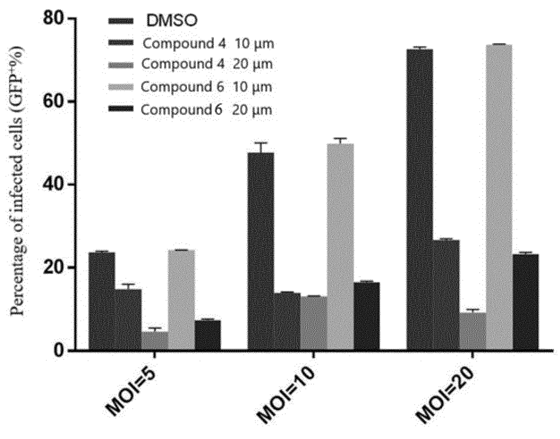


FIG. 7

## Description

### PRIORITY INFORMATION

This application claims priority to and the benefit of Patent Application No. 202011341469.3, submitted with the China National Intellectual Property Administration on November 25, 2020, which is incorporated herein by reference in its entirety.

### FIELD

The present disclosure relates to the field of drug design and pharmaceutical chemistry, and more particularly to a preparation method for and application of a class of stellate bifunctional compounds targeting spike protein against respiratory tract infection virus and salts thereof.

### BACKGROUND

COVID-19 has challenged global health, and because the causative virus is a newly identified virus, no specific drug or vaccine has been approved for therapy. Currently, most efforts have been focused on screening inhibitors against key enzymes in the viral life cycle from known drugs. The published clinical trial results remain to be investigated despite of the special channel for new use of known drugs.

The main ideas of researches of current mainstream antiviral drugs include acquiring target molecules (mostly proteins) involved in viral replication, designing or screening their inhibitors, and clarifying the interaction mode between receptors and ligand molecules to find out specific drugs. Modem technology enables us to quickly obtain structural data of drug targets, and the time to select drug candidates can also be shorten by drug new use strategies. In the field of small molecule drug research, most pharmacologists are looking for specific targeting drugs against SARS-CoV-2, with primary targets including spike proteins (S proteins), E proteins (envelope proteins), M membrane proteins, N nucleocapsid proteins, and various enzymes involved in viral replication of pathogens; and targets of hosts, such as ACE2. However, it is impossible to develop specific targeting drugs for each virus as wild animals carry nearly half a million viruses that may infect humans. The outbreak of the virus epidemic and the high variability of the virus make it difficult for simple targeting drugs to respond to the need for epidemic prevention and control in time, since they are easily rendered ineffective due to the rapid mutation of RNA viruses. In addition to a long development cycle, the major problems for simple antiviral targeting drugs includes: (1) drug resistance develops due to rapid viral mutation; and (2) specific antiviral drugs are no longer effective in critically ill patients in the late stages of infection who are in death risk due to an inflammatory storm or thrombosis.

### SUMMARY

The present disclosure aims to at least solve one of the technical problems in the related art to a certain extent. To this end, one object of the present disclosure is to provide a novel compound that targets spike proteins and has a significant anti-SARS-CoV-2 ability and has a certain broad spectrum. The compounds and salts thereof provided in the present disclosure bind to spike proteins on the surfaces of respiratory tract infection-causing virus particles containing spike proteins at orthotopic and allosteric sites, resulting in failure of the spike proteins on the surfaces of the virus particles in binding to receptor proteins of host cells (e.g., angiotensin converting enzyme 2, or neuraminidase), thereby rendering the incapability of the virus in infecting humans.

To this end, one aspect of the present disclosure provides a compound, or a stereoisomer, a hydrate, a solvate, a pharmaceutically acceptable salt, a metabolite, or a prodrug thereof. According to an embodiment of the present disclosure, the compound has at least one basic unit $R(X)_n$,
wherein,

R is a monosaccharide or a derivative and an aromatic ring thereof, wherein at least one of the monosaccharide or the derivative thereof and the aromatic ring is optionally substituted with at least one substituent selected from the group consisting of amino and carboxyl,
n represents an integer selected from 1 to 6,
each X is linked to a skeleton atom of R, and
the n groups X are identical or different and each independently have a structure $\sim QC(O)\text{-}R'((X')_m)$,
where:

R' represents an optionally substituted six-membered aromatic ring, coumarin, isocoumarin, flavone, isoflavone,

or glucosamine,
m represents an integer selected from 1 to 3,
the m groups X' are identical or different and each independently linked to a ring atom of R',
X' represents QH or QC(O)R",
Q represents -O-, -N-, or $-SO_2-$, and
R" represents a monosaccharide group.

According to the characteristics that coronaviruses (e.g., SARS, MERS, SARS-CoV-2, etc.) and influenza viruses have a common mechanism in virus-host cell membrane fusion, i.e., host cells are infected by the binding of spike proteins on their surfaces to ACE2 enzyme or neuraminidase on surfaces of the host cells, new methods is proposed by the inventors to solve the problems of drug resistance of antiviral targeting drugs:

1) based on the structure of spike protein target, small molecules binding to spike proteins at orthotopic and allosteric sites were designed and screened, so as to combine the advantages of targeting and anti-coronavirus broad spectrum;
2) in order to treat COVID-19 caused by SARS-CoV-2, small anticoagulant molecules that inhibit the spike protein target and prevent thrombosis are sought.

In silico, in vitro, and in vivo experiments have demonstrated that the inventors have inventively discovered small stellate molecules with the above-mentioned dual functions. The small stellate molecule compound has at least one aforementioned basic unit. This class of molecules and salts thereof bind at an orthotopic binding site such as a RBD domain or an allosteric site, to a virus containing spike proteins or a virus with spike proteins on its surface, thereby preventing coronaviruses or other viruses which express spike proteins on their surfaces from invading host cells, and preventing the occurrence of viral infections. In addition, this class of molecules and salts thereof interact with vitamin K-dependent proteins in the human body to inhibit the expression of vitamin K so as to inhibit blood coagulation in the human body, thereby treating thrombosis caused by coronaviruses and producing a curative effect for pneumonia caused by a severe viral infection.

According to an embodiment of the present disclosure, when X' represents QC(O)R", the compound contains a plurality of basic units $R(X)_n$, wherein one of two adjacent basic units $R(X)_n$ is linked to a carbon or oxygen atom of a skeleton of R in another one of the two adjacent basic units $R(X)_n$ through QC(O) in X', and a recursive hierarchy of the plurality of basic units $R(X)_n$ in the compound includes not more than 2 levels.

According to an embodiment of the present disclosure, when X' represents QC(O)R", R" represents a monosaccharide group, the compound contains a plurality of basic units $R(X)_n$, wherein one of two adjacent basic units $R(X)_n$ is linked to a carbon atom or atom of R in another one of the two adjacent basic units $R(X)_n$ through the monosaccharide group in X', and a recursive hierarchy of each of the plurality of basic units $R(X)_n$ in the compound includes not more than 2 levels.

A class of stellate molecules (including a plurality of said basic units) and salts thereof was designed creatively by the inventors of the present disclosure, with viruses that contain spike proteins and cause pulmonary infections (such as coronaviruses and influenza viruses) as an object, and the spike proteins as a target. Such spike proteins invade host cells. Molecular dynamics simulation was used to screen natural product-like stellate molecules capable of multipoint docking with the spike proteins at orthotopic or allosteric sites to mediate the formation of homologous or heterologous *k*-polymers ($k$>1) of the spike proteins. A small molecule library was prepared by chemical synthesis, and each kind of molecules was subjected to a molecular dynamics simulation experiment with a time course of 100 nanoseconds to select lead compounds. These lead compounds were subjected to experimental verification at a cellular level and fluorescence probes showed that this class of molecules and salts thereof did prevent the invasion of virus particles into host cells. Metabolites of these compounds and salts thereof in the body further inhibited the expression level of vitamin K, thereby treating thrombosis caused by invasion of such viruses into pulmonary host cells.

According to an embodiment of the present disclosure, R is the monosaccharide or the derivative thereof or the aromatic ring. The monosaccharide or the derivative thereof or the aromatic ring is optionally substituted with at least one substituent selected from the group consisting of amino and carboxyl;

R' represents the optionally substituted six-membered aromatic ring, coumarin, flavone, or isoflavone; and
Q represents -O- and -N-.

According to an embodiment of the present disclosure, the monosaccharide or the derivative thereof is selected from glucose, gluconic acid, or glucosaminic acid, and the aromatic ring is selected from $C_4$-$C_{20}$ aromatic rings.

According to an embodiment of the present disclosure, the aromatic ring is selected from phenyl, chromone, or isoflavone.

According to an embodiment of the present disclosure, R' represents the optionally substituted six-membered aromatic ring, coumarin, or flavone; and
Q represents -O-.

According to an embodiment of the present disclosure, the basic unit in the compound is one selected from the following structures:

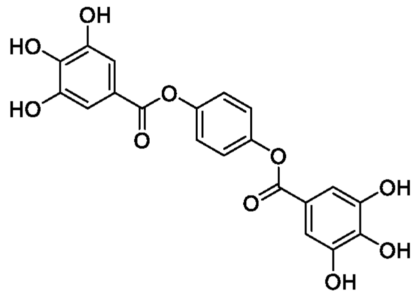

Compound 1

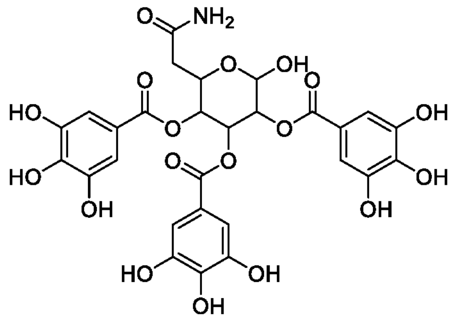

Compound 2

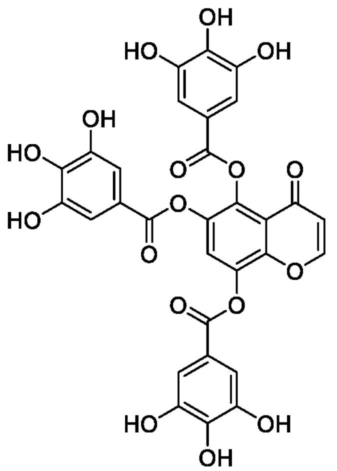

Compound 3

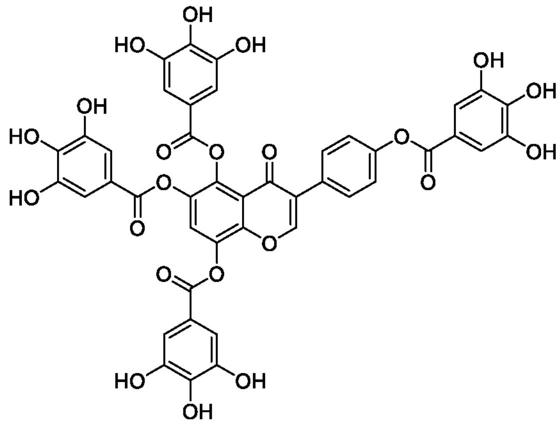

Compound 4

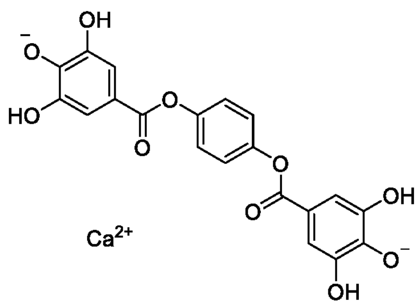

Compound 5

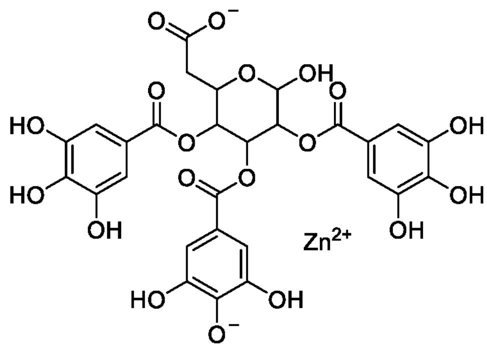

Compound 6

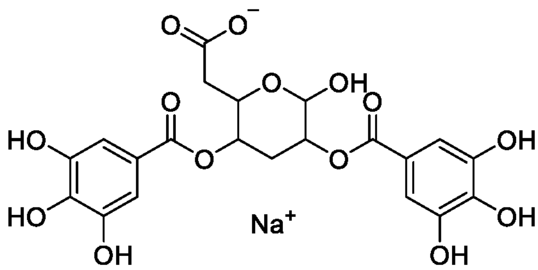

Compound 7.

Compound 1: when R is phenyl, n is equal to 2, and groups X are in the para positions; Q in group X is O, m in R' is equal to 3, the groups X' is in the meta and para positions, and X' is hydroxyl group.
Compound 2: R is a glucosaminic acid skeleton, n is equal to 3, and the groups X are in position 2, 3, and 4 of the glucosaminic acid skeleton; Q in group X is O, m in R' is equal to 3, groups X' are in the meta and para positions, and X' is hydroxyl.
Compound 3: R is chromone, n is equal to 3, and groups X are in the positions 5, 6, and 8 of the chromone; Q in group X is O, m in R' is equal to 3, groups X' are in the meta and para positions, and X' is hydroxyl.
Compound 4: R is isoflavone, n is equal to 4, and groups X are in the position 5, 6, 8, and 4' of the isoflavone; Q in group X is O, m in R' is equal to 3, groups X' are in the meta and para positions, and X' is hydroxyl.
Compound 5: a divalent calcium salt of Compound 1.
Compound 6: R is a gluconic acid skeleton, n is equal to 3, and groups X are in position 2, 3, and 4 of the gluconic acid amine skeleton; Q in group X is O, m in R' is equal to 3, groups X' are in the meta and para positions, and X' is hydroxyl; and carboxyl of the compound forms a salt with zinc.
Compound 7: a metabolite of Compound 6. In Compound 7, R is a 3-deoxygluconic acid skeleton, n is equal to 2, and groups X are in the positions 2 and 4 of the 3-deoxygluconic acid skeleton; Q in group X is O, m in R' is equal to 3, groups X' are in the meta and para positions, and X' is hydroxyl; and carboxyl of the compound forms a salt with sodium.
In a second aspect of the present disclosure, provided is a pharmaceutical composition. According to an embodiment of the present disclosure, the pharmaceutical composition includes the compound, or the stereoisomer, the hydrate, the solvate, the pharmaceutically acceptable salt, the metabolite, or the prodrug thereof according to the first aspect.
The pharmaceutical composition provided by the present disclosure can be used for treating or preventing diseases caused by viral infections in host cells, wherein the viruses have spike proteins on surfaces thereof, and the pharmaceutical composition of the present disclosure can inhibit the increase of vitamin K and inhibit pulmonary thrombosis.
According to an embodiment of the present disclosure, the pharmaceutical composition is in a dosage form selected from powders, tablets, granules, capsules, solutions, emulsions, suspensions, injections, sprays, aerosols, or dry powder inhalations, preferably nasal sprays.
In a third aspect of the present disclosure, provided is the use of the compound, or the stereoisomer, the hydrate, the solvate, the pharmaceutically acceptable salt, the metabolite, or the prodrug thereof in the manufacture of drugs for at least one of:

treating or preventing diseases caused by infections of viruses in host cells, preferably the viruses having spike proteins on surfaces thereof;
inhibiting the increase of vitamin K; and
inhibiting pulmonary thrombosis.

According to an embodiment of the present disclosure, the viruses include coronaviruses and influenza viruses.
According to an embodiment of the present disclosure, the coronaviruses include SARS, MERS, and SARS-CoV-2.
According to an embodiment of the present disclosure, the diseases include COVID-19, influenza A, and influenza B.
A second object of the present disclosure is to provide a compound having a therapeutic effect on COVID-19.
A third object of the present disclosure is to provide a method for the preparation of the bifunctional compounds and salts thereof.
The bifunctional compounds refer to those that prevent the invasion of host cells by virus particles, and the

metabolites of these compounds and salts thereof in the body further inhibit the expression level of vitamin K, thereby treating thrombosis caused by invasion of such viruses into pulmonary host cells.

In a fourth aspect of the present disclosure, provided is the use of a drug in the prevention and/or treatment of diseases caused by infections of viruses in host cells. According to an embodiment of the present disclosure, the drug includes the compound, or the stereoisomer, the hydrate, the solvate, the pharmaceutically acceptable salt, the metabolite, or the prodrug thereof according to the first aspect and/or the pharmaceutical composition according to the second aspect. Here, the viruses have spike proteins on surfaces thereof, and the diseases include COVID-19, influenza A, and influenza B.

According to an embodiment of the present disclosure, the viruses include coronaviruses and influenza viruses.

According to an embodiment of the present disclosure, the coronaviruses include SARS, MERS, and SARS-CoV-2.

In a fifth aspect of the present disclosure, provided is the use of a drug in the reduction of a vitamin K level in a patient with increased vitamin K. According to an embodiment of the present disclosure, the drug includes the compound, or the stereoisomer, the hydrate, the solvate, the pharmaceutically acceptable salt, the metabolite, or the prodrug thereof according to the first aspect and/or the pharmaceutical composition according to the second aspect.

In a sixth aspect of the present disclosure, provided is the use of a drug in the prevention and/or treatment of pulmonary thrombosis. According to an embodiment of the present disclosure, the drug includes the compound, or the stereoisomer, the hydrate, the solvate, the pharmaceutically acceptable salt, the metabolite, or the prodrug thereof according to the first aspect and/or the pharmaceutical composition according to the second aspect.

In a seventh aspect of the present disclosure, provided is a method for preventing and/or treating diseases caused by infections of viruses in host cells. According to an embodiment of the present disclosure, the method includes administering the pharmaceutical composition of the second aspect to a patient suffering from or suspected of suffering from the diseases caused by the infections of the viruses in the host cells. Here, the viruses have spike proteins on surfaces thereof.

According to an embodiment of the present disclosure, the diseases include COVID-19, influenza A, and influenza B.

According to an embodiment of the present disclosure, the viruses include coronaviruses and influenza viruses.

According to an embodiment of the present disclosure, the coronaviruses include SARS, MERS, and SARS-CoV-2.

In an eighth aspect of the present disclosure, provided is a method for the reduction of a vitamin K level. According to an embodiment of the present disclosure, the method includes administering the pharmaceutical composition of the second aspect to a patient in need of reducing the vitamin K level.

In a ninth aspect of the present disclosure, provided is a method for preventing and/or treating pulmonary thrombosis. According to an embodiment of the present disclosure, the method includes administering the pharmaceutical composition of the second aspect to a patient suffering from or suspected of suffering from the pulmonary thrombosis.

Compared with the prior art, the technical advancements of the present disclosure are summarized as follows:

1. The lead compounds (stellate molecules) of the present disclosure are pathogen-targeting drugs (the target is the spike protein) with good pathogen specificity; also due to the binding not only to the RBD domain of the spike protein but also to multiple sites of the spike protein, they have a broad spectrum activity against coronaviruses and influenza viruses, which is a characteristic that other antiviral drugs hardly possess;
2. The lead compounds of the present disclosure are metabolized in vivo to coumarin (warfarin analogues) natural anticoagulant products after completing the antiviral mission, and thus have therapeutic effects on Severe COVID-19; many antiviral compounds have only prophylactic effects; however, the compounds provided by the present disclosure have both prophylactic effects and therapeutic effects;
3. The lead compound molecules of the present disclosure are designed according to the structural features of the spike proteins, then virtually screened by molecular dynamics simulation technology based on super-computation, and finally determined by biological experimental verification;
4. The lead compound with the best activity provided by the present disclosure is derived from "new use of known drugs" of natural products, and historically, the lead compound was used as "universal antidote" together with activated carbon and magnesium oxide, and can be administered orally and externally for the treatment of cold sores and fever blisters, diaper rash, miliaria, sore throat, tonsil swelling and pain, erythra, chronic diarrhea, dysentery, hematuria, arthralgia, and persistent cough and cancer, and also used as a flavoring agent in foods and beverages, thus having high clinical safety;
5. The lead compounds of the present disclosure are widely distributed in medicinal plants and abundant in resources, which is easy to modify the activity through chemical synthesis, so as to further improve the drugability.

## BRIEF DESCRIPTION OF DRAWINGS

The foregoing and/or additional aspects and advantages of the present disclosure will become apparent and readily appreciated from the following description of the embodiments taken in conjunction with the accompanying drawings, in which:

FIGS. 1 to 6 show preparation schemes for the synthesis of compounds 1 to 6 in some embodiments provided in the present disclosure;
FIG. 7 shows the inhibitory activity of compounds 4 and 6 against virus infection of host cells at three different virus titers, wherein the bar graphs represent, from left to right, the percentage of infected cells after treatment with DMSO, 10 μL of compound 4, 20 μL of compound 4, 10 μL of compound 5, and 20 μL of compound 5, respectively, at each virus titer;
FIG. 8 shows the results of transmission electron microscopy experiments on compounds 4 and 6 inhibiting virus infection of host cells at three different virus titers. Experimental cells: A549; time of virus infection: 48 h; virus titer: 10^9 TU/mL; number of plated cells: 5*10^4/well; virus amount: MOI=20(1*10^6TU);
FIG. 9 shows changes in fluorescence intensity of virus within host cells with virus infection inhibited by compounds 4 and 6 at three different virus titers; and
FIG. 10 shows the anticoagulant activity of metabolite of compound 6.

## DESCRIPTION OF EMBODIMENTS

The present disclosure will be further described with reference to specific examples and drawings, but should not be construed as being limited thereto. Without departing from the ideas and essence of the present disclosure, simple modifications or replacements to the methods, steps, or conditions of the present disclosure are within the scope of the present disclosure. Unless otherwise specified, the technical means used in the examples are conventional means well known to those skilled in the art.

### Terms

The term "include" is intended to be open-ended, i.e., to include what is specified in the present disclosure, without excluding contents of other aspects.

"Stereoisomer" refer to a compound that has the same chemical structure, but has a different spatial arrangement of atoms or groups. Stereoisomers include enantiomers, diastereomers, conformational isomers (rotamers), geometric isomers, (cis/trans) isomers, atropisomers, and the like.

"Solvate" of the present disclosure refers to an association compound of one or more solvent molecules with a compound of the present disclosure. Solvents for the formation of solvates include, but are not limited to water, isopropanol, ethanol, methanol, dimethyl sulfoxide, ethyl acetate, acetic acid, and aminoethanol. The term "hydrate" refers to an association compound formed with water as the solvent.

As used herein, the term "pharmaceutically acceptable salt" refers to those salts which are, within the scope of reasonable medical judgment, suitable for use in contact with the tissues of human and animals without excessive toxicity, irritation, allergic response, or the like, and are commensurate with a reasonable benefit/risk ratio. The pharmaceutically acceptable salts are well known in the art. For example, the pharmaceutically acceptable salts are described in detail by S.M.Berge, et al., J.Pharm.Sci.66:1-19, 1977. These salts can be prepared in situ during the final isolation and purification of the compounds of the disclosure, or prepared separately by reacting a free base group with a suitable organic acid. Representative acid addition salts include acetate, adipate, alginate, ascorbate, aspartate, benzene sulfonate, benzoate, bisulfate, borate, butyrate, camphorate, camsylate, carbonate, chloride, citrate, cypionate, digluconate, dodecyl sulfate, ethanesulfonate, fumarate, glucoheptonate, glycerophosphate, hemisulfate, heptanoate, caproate, hydrobromide, hydrochloride, hydroiodide, 2-hydroxyethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectate, persulfate, 3-phenylpropionate, phosphate, picrate, pivalate, propionate, stearate, succinate, sulfate, tartrate, thiocyanate, tosylate, undecanoate, valerate, etc. Representative alkali or alkaline earth metal salts include salts of sodium, lithium, potassium, calcium, magnesium, etc., as well as nontoxic ammonium, quaternary ammonium, and amine cations, including, but not limited to ammonium, tetramethylammonium, tetraethylammonium, methylamine, dimethylamine, trimethylamine, triethylamine, ethylamine, etc.

"Prodrug" is a pharmacological substance which is administered in an inactive (or significantly less active) form and is subsequently metabolized to an active metabolite in the body. The fundamental principles behind prodrugs is generally used to bring more drugs to the desired target at lower doses, which is generally attributed to better absorption, distribution, metabolism, and/or excretion (ADME) properties. The prodrug is often designed to improve oral bioavailability

as poor gastrointestinal absorption is usually the limiting factor. In addition, the use of prodrug strategies may increase the selectivity of a drug for its intended target, thereby reducing the likelihood of off-target effects.

"One of two adjacent basic units is linked to a skeleton atom of R in another one of the two adjacent basic units through a monosaccharide group in X'" means that the two adjacent basic units are connected by esterification between the hydroxyl group in the monosaccharide group in X' in one of the two adjacent basic units and the carboxyl group on the skeleton atom of R in the other basic unit.

"The recursive hierarchy of the basic units in the compound includes not more than 2 levels".

The drug of the present disclosure may be prepared in various dosage forms including, but not limited to powders, tablets, granules, capsules, solutions, emulsions, suspensions, injections, sprays, aerosols, or dry powder inhalations, preferably nasal sprays.

The drug of the present disclosure may employ various pharmaceutically acceptable adjuvants/excipients, including but not limited to solvents, solubilizers, binders, stabilizers, antioxidants, pH modifiers, flavoring agents, etc., depending on the requirements and the common knowledge in the pharmaceutical field.

According to some specific embodiments of the present disclosure, provided is a class of stellate compounds and salts thereof targeting spike proteins, the compounds having s structure of:

$$R(X)_n,$$

where n =1 to 6,

wherein R is a monosaccharide or an aminomonosaccharide or a monosaccharide carboxyl, or a skeleton of an aromatic ring. $X_{1-n}$ is a plurality of substituents on the R skeleton, and $X_i$ may be the same as or different from $X_{i+1}$, but the total number of substituents on R is not more than 6.

The structure of X is shown below:

$$\sim QC(O)\text{-}R'((X')_m),$$

where m =1 to 3,

R'= {a six-membered aromatic ring, coumarin, flavone, isoflavone, glucosamine};
Q={-O-, -N-, -$SO_2$-}, X'={QH, QC(O)R"}
R"= {$R(X)_n$, a monosaccharide group}

These molecules and salts thereof bind to spike proteins on the surfaces of respiratory tract infection-causing virus particles containing spike proteins at orthotopic and allosteric sites, resulting in failure of the spike proteins on the surfaces of the virus particles in binding to receptor proteins of host cells (e.g., angiotensin-converting enzyme 2, or neuraminidase), thereby rendering the virus incapable of infecting humans.

According to some specific embodiments of the present disclosure, R is a monosaccharide or an aminomonosaccharide or a monosaccharide carboxyl, or a skeleton of an aromatic ring. $X_{1-n}$ is a plurality of substituents on the R skeleton, and $X_i$ may be the same as or different from $X_{i+1}$, but the total number of substituents on R is not more than 6. The structure of X is defined as ~QC(O)-R'($(X')_m$), where m = 1 to 3; R' is defined as {a six-membered aromatic ring, coumarin, isocoumarin, flavone, and isoflavone}; Q={-O-, -N-}, X'={QH, QC(O)R"}; R" is defined as {$R(X)_n$, a monosaccharide group}, n = 1 to 6.

Such molecules and salts thereof decompose into metabolites in the human body, including 3,4,5-trihydroxybenzoic acid glucoside polymers, polyhydroxy coumarin glycoside polymers, polyhydroxy flavone glycoside polymers; such metabolites inhibit the expression level of vitamin K in human body, prevent platelet aggregation, and thus treat thrombosis caused by coronaviruses.

According to some specific embodiments of the present disclosure, R is a monosaccharide or an aminomonosaccharide or a monosaccharide carboxyl, or a skeleton of an aromatic ring. $X_{1-n}$ is a plurality of substituents on the R skeleton, and $X_i$ may be the same as or different from $X_{i+1}$, but the total number of substituents on R is not more than 6. The structure of X is defined as ~QC(O)-R'($(X')_m$), where m = 1 to 3; R' is defined as {a six-membered aromatic ring, coumarin, flavone}; Q={-O-, -N-}, X'={QH, QC(O)R"}; R" is defined as {$R(X)_n$, a monosaccharide group}, n = 1 to 6. A method for the preparation of this class of compounds is characterized in that polyhydroxybenzoic acid is esterified with monosaccharide at room temperature, and then condensed with polyhydroxybenzoic acid, flavone, or coumarin to obtain stellate molecules. Such molecules and salts thereof bind at an orthotopic binding site, such as a RBD domain or an allosteric site, to SARS-CoV-2 or viruses with spike proteins on their surfaces, thereby preventing coronaviruses or other viruses which express spike proteins on their surfaces from invading host cells, and preventing the occurrence of viral

infections.

According to some specific embodiments of the present disclosure, R is a monosaccharide or an aminomonosaccharide or a monosaccharide carboxyl, or a skeleton of an aromatic ring. $X_{1-n}$ is a plurality of substituents on the R skeleton, and $X_i$ may be the same as or different from $X_{i+1}$, but the total number of substituents on R is not more than 6. The structure of X is defined as ~QC(O)-R'((X')$_m$), where m = 1 to 3; R' is defined as {a six-membered aromatic ring, coumarin, flavone}; Q={-O-, -N-}, X'={QH, QC(O)R"}; R" is defined as {R(X)$_n$, a monosaccharide group}, where n = 1 to 6. A method for the preparation of this class of compounds is characterized in that polyhydroxybenzoic acid is esterified with monosaccharide at room temperature, and then condensed with polyhydroxybenzoic acid, flavone, or coumarin to obtain stellate molecules. Such molecules and salts thereof bind at an orthotopic binding site, such as a RBD domain or an allosteric site, to viruses containing spike proteins or viruses with spike proteins on their surface, thereby preventing coronaviruses or other viruses which express spike proteins on their surfaces from invading host cells, and preventing the occurrence of viral infections.

According to some specific embodiments of the present disclosure, $R_1$ is a monosaccharide or an aminomonosaccharide or a monosaccharide carboxyl, or an aromatic ring. $X_{1-n}$ is a plurality of substituents on the R skeleton, and $X_i$ may be the same as or different from $X_{i+1}$, but the total number of substituents on R is not more than 6. The structure of X is defined as ~QC(O)-R'((X')$_m$), where m = 1 to 3; R' is defined as {a six-membered aromatic ring, coumarin, flavone}; Q={-O-, -N-}, X'={QH, QC(O)R"}; R" is defined as {R(X)$_n$, a monosaccharide radical}, where n = 1 to 6. According to a method for preparing this class of compounds, polyhydroxy benzoic acid is esterified with monosaccharide at room temperature, and then condensed with polyhydroxy benzoic acid, flavone, or coumarins to obtain stellate molecules. These molecules and salts thereof interact with vitamin K-dependent proteins in the human body to inhibit the expression of vitamin K so as to inhibit blood coagulation in the human body, thereby treating thrombosis caused by a coronavirus and producing a curative effect for pneumonia caused by a severe viral infection.

According to some specific examples of the present disclosure, typical stellate compounds are embodied as follows:

R(X)$_n$:

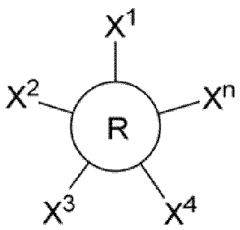

n = 1 - 6

R = 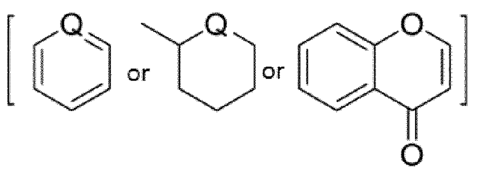

X = [ —QC(O)—R' ] or QH

Q = -N-, -SO$_2$-, -O-

R' = 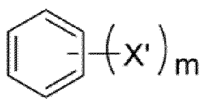

X' = [ —QC(O)—R" ] or QH

Q = O

R" = 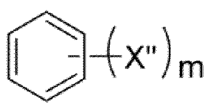

X" = OH

According to some embodiments of the present disclosure, provided is a method for the preparation of a stellate bifunctional compound targeting spine protein-containing viruses causing pulmonary infections, including the following step:

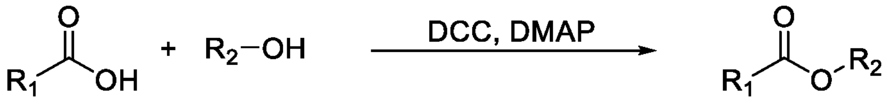


A synthetic scheme for the stellate bifunctional compounds targeting spine protein-containing viruses causing pulmonary infections is shown above. To dichloromethane (DMF, pyridine, tetrahydrofuran, etc. may be used instead) was added phenolic starting materials containing the carboxylic acid group ($R_1$-COOH), and were added dicyclohexylcarbodiimide (DCC) and 4-dimethylaminopyridine (DMAP) simultaneously. The mixture was stirred at room temperature for 0.5 h. Saccharide with or without protecting groups, aromatic rings with hydroxyl groups, aliphatic chains with hydroxyl groups ($R_2$-OH) were then added, and the mixture was stirred for 8 h at room temperature or under heating. The mixture was extracted with ethyl acetate or dichloromethane, and the organic layer was subjected to spin-drying to remove the organic solvent, and separation and purification by column chromatography to obtain the desired molecules.

The Examples of the present disclosure will be described in detail below. The examples described below are exemplary, and are only used to explain the present disclosure but should not be construed as a limitation to the present disclosure. Examples, where specific techniques or conditions are not specified, are implemented in accordance with techniques or conditions described in the literatures in the art or according to the product description. All of the used agents or instruments which are not specified with the manufacturer are conventional and commercially-available products.

**Example 1 Synthesis of Compound 1**

3,4,5-tribenzyloxybenzoic acid (2.2 mmol), dicyclohexylcarbodiimide (DCC, 2.2 mmol), and 4-dimethylaminopyridine (DMAP, 0.2 mmol) were added to dichloromethane while being stirred for 0.5 h at room temperature; then 1,4-benzenediol (1 mmol) was added, and the mixture was stirred at room temperature for 8 h. Then, the mixture was extracted with ethyl acetate, and the organic layer was subjected to spin-drying to remove the organic solvent, and separation and purification by column chromatography to obtain an intermediate. The intermediate and palladium on carbon (60 mg) were added to methanol, air was suctioned out, and then hydrogen was filled; and the mixture was stirred at room temperature for 2 h. The palladium on carbon was removed by filtration and the organic layer was spin-dried. Column chromatography was conducted to give compound 1 with an overall yield of 62.4%. The synthesis scheme of compound 1 is shown in FIG. 1.

**Example 2 Synthesis of Compound 2**

3,4,5-tribenzyloxybenzoic acid (3.3 mmol), dicyclohexylcarbodiimide (DCC, 3.3 mmol), and 4-dimethylaminopyridine (DMAP, 0.3 mmol) were added to dichloromethane while being stirred for 0.5 h at room temperature. Then 2-(6-(benzyloxy)-3,4,5-trihydroxytetrahydro-2H-pyran-2-yl)acetamide (1 mmol) was added and the mixture was stirred at room temperature for 8 h. Then the mixture was extracted with ethyl acetate, the organic layer was subjected to spin-drying to remove the organic solvent, and separation and purification by column chromatography to obtain an intermediate. The intermediate and palladium on carbon (100 mg) were added to methanol, air was suctioned out and then hydrogen was filled; and the mixture was stirred at room temperature for 2 h. The palladium on carbon was removed by filtration and the organic layer was spin-dried. Column chromatography was conducted to give compound 2 with an overall yield of 53.5%. The synthesis scheme of compound 2 is shown in FIG. 2.

**Example 3 Synthesis of Compound 3**

3,4,5-tribenzyloxybenzoic acid (3.3 mmol), dicyclohexylcarbodiimide (DCC, 3.3 mmol), and 4-dimethylaminopyridine (DMAP, 0.3 mmol) were added to dichloromethane while being stirred for 0.5 h at room temperature. Then 5,6,8-trihydroxy-4H-chromen-4-one (1 mmol) was added and the mixture was stirred at room temperature for 8 h. Then the mixture was extracted with ethyl acetate, and the organic layer was subjected to spin-drying to remove the organic solvent, and separation and purification by column chromatography to obtain an intermediate. The intermediate and palladium on carbon (100 mg) were added to methanol, air was suctioned out, and then hydrogen was filled; and the mixture was stirred at room temperature for 2 h. The palladium on carbon was removed by filtration and the organic layer was spin-dried. Column chromatography was conducted to give compound 3 with an overall yield of 61.2%. The synthesis scheme of compound 3 is shown in FIG. 3.

**Example 4 Synthesis of Compound 4**

3,4,5-tribenzyloxybenzoic acid (4.4 mmol), dicyclohexylcarbodiimide (DCC, 4.4 mmol), and 4-dimethylaminopyridine (DMAP, 0.4 mmol) were added to dichloromethane while being stirred for 0.5 h at room temperature; Then 5,6,8-trihydroxy-3-(4-hydroxyphenyl)-4H-chromen-4-one (1 mmol) was added and the mixture was stirred at room temperature for 8 h. Then the mixture was extracted with ethyl acetate, and the organic layer was subjected to spin-drying to remove the organic solvent, and separation and purification by column chromatography to obtain an intermediate. The intermediate and palladium on carbon (120mg) were added to methanol, air was suctioned out, and then hydrogen was filled; and the mixture was stirred at room temperature for 2 h. The palladium on carbon was removed by filtration and the organic layer was spin-dried. Column chromatography was conducted to give compound 4 with an overall yield of 76.3%. The synthesis scheme of compound 4 is shown in FIG. 4.

**Example 5 Synthesis of Compound 5**

Compound 1 (1 mmol) and calcium bicarbonate (1 mmol) were added to tert-butanol and the mixture was stirred at room temperature for 12 h, followed by spin-drying to remove the solvent, to give the desired compound 5 with an overall yield of 98.9%. The synthesis scheme of compound 5 is shown in FIG. 5.

**Example 6 Synthesis of Compound 6**

3,4,5-tribenzyloxybenzoic acid (3.3 mmol), dicyclohexylcarbodiimide (DCC, 3.3 mmol), and 4-dimethylaminopyridine (DMAP, 0.3 mmol) were added to dichloromethane while being stirred for 0.5 h at room temperature. Then 2-(6-(benzyloxy)-3,4,5-trihydroxytetrahydro-2H-pyran-2-yl)acetic acid (1 mmol) was added and the mixture was stirred at room temperature for 8 h. Then the mixture was extracted with ethyl acetate, and the organic layer was subjected to spin-drying to remove the organic solvent, and separation and purification by column chromatography to obtain an intermediate. The intermediate and palladium on carbon (100 mg) were added to methanol, air was suctioned out, and then hydrogen was filled; and the mixture was stirred at room temperature for 2 h. The palladium on carbon was removed by filtration and the organic layer was spin-dried. Column chromatography was conducted to give another intermediate b. The intermediate b and zinc hydroxide were added to tert-butanol, the mixture was stirred at room temperature for 2 h, followed by spin-drying to remove the solvent, to give compound 6 with an overall yield of 49.8%. The synthesis scheme of compound 6 is shown in FIG. 6.

**Example 7 Anti-coronavirus invasion experiment of compounds obtained in Examples 1 to 6**

**Experimental** Materials and Reagents

H1299 Human lung cancer cells, cultured with RPMI-1640 10% fetal bovine serum in an incubator with 5% $CO_2$ and saturated humidity at 37°C.
RPMI-1640 + 10% Fetal Bovine Serum
PBS (Life Science Products&Services)
Trypsin-EDTA Solution (Gibco)
24-well plate (Corning)
Lentivirus-NC virus solution (GenePharma, $1\times10^9$ TU/mL)

Principles and procedures for virus infection experiment

1. H1299 cells in good condition were digested and then re-suspended, and an appropriate amount of cells were inoculated into a 24-well plate and was incubated in an incubator at 37°C overnight;
2. negative control viruses were mixed and diluted with 1640 culture medium according to MOI of 5, 10, and 20, with a total volume of about 500 μL, and Polybrene was added with a final concentration of 5 μg/mL;
3. the original culture medium was pipetted away from the 24-well plate, the negative control virus gradient diluted solution was added instead, and the mixture was incubated in an incubator at 37°C;
4. the negative control virus diluted solution was pipetted away after 24 h and replaced with 500 μL of fresh culture medium, and the mixture was incubated in an incubator at 37°C;

5. the plate was observed under a fluorescent Inverted microscope after 48 h and the results were recorded, with the results shown in FIGS. 7 to 9.

The results were shown in FIGS. 7 to 9: FIG. 7 shows the inhibitory activity of compounds 4 and 6 against virus infection of host cells at three different virus titers, wherein the bar graphs represent, from left to right, the percentage of infected cells after treatment with DMSO, 10 μL of compound 4, 20 μL of compound 4, 10 μL of compound 6, and 20 μL of compound 5, respectively, at each virus titer; FIG. 8 shows the results of transmission electron microscopy experiments on compounds 4 and 6 inhibiting virus infection of host cells at three different virus titers. Experimental cells: A549; time of virus infection: 48 h; virus titer: 10^9 TU/mL; number of plated cells: 5*10^4/well; virus amount: MOI=20(1*10^6TU); FIG. 9 shows changes in fluorescence intensity of virus within host cells with virus infection inhibited by compounds 4 and 6 at three different virus titers; and

FIGS. 7 and 9 show that at different virus titers, stellate compounds 4 and 6 at 20 μM concentration could significantly inhibit virus infection in cells; and stellate compound 4 also significantly inhibited virus infection in cells at 10 μM concentration.

**Example 8 Anticoagulation experiment study of metabolites of compounds obtained in Examples 1 to 6**

1. Principle for experiments

Coagulation is a complex process involving a series of coagulation factors, which essentially include two pathways, "intrinsic coagulation pathway" and "extrinsic coagulation pathway". Activated partial thromboplastin time (APTT), prothrombin time (PT), and thrombin time (TT) are adopted usually in the laboratory to evaluate the anticoagulant effect.

2. Preparation of reagents

2.1 Preparation of anticoagulation experiment reagents

APTT kit, PT kit, and TT kit, preheated for 2 min at 37°C before use.

3. Procedures

3.1 Anticoagulation experiment procedures

3.1.1 Preparation of plasma

New Zealand rabbits were anaesthetized with chloral hydrate, heart puncture was conducted to collect blood into an anticoagulant tube containing 1/10 vol 3.8% sodium citrate, followed by centrifuging at 3000 rpm for 15 min, supernatant was collected to obtain platelet poor plasma which was stored in a refrigerator for later use.

3.1.2 Preparation of samples to be tested

The compounds were prepared with DMSO to obtain stock solutions with a concentration of 10 mM. Samples to be tested were diluted with normal saline during the test, with a final concentration of 50 μM, 12.5 μM, 3.13 μM, 0.78 μM, 0.20 μM, and 0.05 μM. The sample to be tested at each concentration had a volume of 50 μL, with normal saline solution of the same volume as a negative control.

3.1.3 Test method

APTT Assay: 100 μL of plasma was mixed well with 50 μL of samples to tested respectively, the mixture was pre-heated at 37°C for 2 min, then 50 μL of APTT reagent was added, followed by incubation at 37°C for 3 min, addition of 50 μL of 0.025 mol/L $CaCl_2$, and detection on an automatic blood coagulation analyzer to record the coagulation time.

PT Assay: 50 μL of plasma was mixed well with 20 μL of samples to be tested respectively, the mixture was pre-heated at 37°C for 2 min, followed by incubation at 37°C for 3 min, and addition of 100 μL of PT reagent. The mixture was mixed well and tested to record the coagulation time.

TT Assay: 100 μL of prepared plasma was mixed well with 50 μL of the sample mixture to be tested, and pre-heated at 37°C for 2 min, followed by addition of 50 μL of TT reagent to the mixture and detection to record results of the instrument.

3.1.4 Statistical methods

One-way analysis of variance (One-Way ANOVA) was performed for multi-group comparison; pairwise comparison for means among groups was performed, with Dunnett's multiple comparisons test in case of homogeneity of variance, and Dunnett's T3 multiple comparisons test in case of heterogeneity of variance. P <0.05 means statistical significance. ****$p$<0.0001, ***$p$<0.001, **$p$<0.01, *$p$<0.05.

The in vitro effects of Compound 6 on the anti-coagulation indexes APTT, PT, and TT were tested using the above methods, and the results are shown in Table 1 and FIG. 10.

In the evaluation of coagulation indexes APTT, PT, and TT of compound 6 at different dose gradients of 50.00 μL, 12.50 μL, 3.13 μL, 0.78 μL, 0.20 μL, and 0.05 μL, stellate compound 6 could dose-dependently inhibit plasma coagulation.

Table 1 In vitro effect of compound 6 on APTT, PT, and TT ($\bar{x}\pm s$, n = 3)

| Group | | APTT/s | PT/s | TT/s |
|---|---|---|---|---|
| Normal saline | | 21.24±1.04 | 10.96±0.39 | 23.00±0.10 |
| Compound 6 | 50.00 (μM) | 106.51±0.94**** | 16.66±0.45*** | 38.87±0.77** |
| Compound 6 | 12.50 (μM) | 75.11±2.58*** | 13.73±0.17* | 28.89±0.68* |
| Compound 6 | 3.13 (μM) | 37.72±1.83** | 12.83±0.19* | 25.25±0.27** |
| Compound 6 | 0.78 (μM) | 26.84±0.79* | 10.54±0.37 | 23.95±0.07** |
| Compound 6 | 0.20 (μM) | 24.03±1.05 | 10.11±0.30 | 22.90±0.09 |
| Compound 6 | 0.05 (μM) | 20.12±0.09 | 9.76±0.85 | 23.35±0.31 |
| Notes: compared to normal saline group, ****$p$<0.0001, ***$p$<0.001, **$p$<0.01, *$p$<0.05. | | | | |

**Example 9 External pharmaceutics experiment study of compounds obtained in Examples 1 to 6**

To 0.4 mg to 17.0 mg of the final products described in FIGS. 1 to 6 were conventionally added 0.85 g to 0.9 g of sodium chloride and 1.0 g to 1.9 mg of citric acid, and 100 ml of distilled water was injected conventionally to obtain a nasal spray according to a conventional nasal spray preparation method.

In addition, description with reference to the term "one embodiment", "some embodiments", "an example", "a specific example", "some examples" or the like means that a specific feature, structure, material, or characteristic described in combination with the embodiment(s) or example(s) are included in at least one embodiment or example of the present disclosure. In this specification, illustrative expressions of these terms do not necessarily refer to the same embodiment or example. Moreover, the specific feature, structure, material, or characteristic described may be combined in any suitable manner in any one or more embodiments or examples. In addition, without mutual contradiction, those skilled in the art may incorporate and combine different embodiments or examples and features of the different embodiments or examples described in this specification.

Although the embodiments of the present disclosure have been illustrated and described, it should be understood that the above embodiments are exemplary and should not be construed as limiting the present disclosure, and persons of ordinary skill in the art may make various changes, modifications, replacements, and variations to the above embodiments without departing from the scope of the present disclosure.

**Claims**

**1.** A compound, or a stereoisomer, a hydrate, a solvate, a pharmaceutically acceptable salt, a metabolite, or a prodrug thereof, the compound having at least one basic unit $R(X)_n$, wherein:

R is a monosaccharide or a derivative thereof or an aromatic ring, wherein at least one of the monosaccharide or the derivative thereof and the aromatic ring is optionally substituted with at least one substituent selected from the group consisting of amino and carboxyl,
n represents an integer selected from 1 to 6,
each X is linked to a skeleton atom of R, and
the n groups X are identical or different and each independently have a structure ~QC(O)-R'($(X')_m$),

where:

R’ represents an optionally substituted six-membered aromatic ring, coumarin, isocoumarin, flavone, isoflavone, or glucosamine,
m represents an integer selected from 1 to 3,
the m groups X’ are identical or different and each independently linked to a ring atom of R’,
X’ represents QH or QC(O)R",
Q represents -O-, -N-, or $-SO_2-$, and
R" represents a monosaccharide group.

2. The compound, or the stereoisomer, the hydrate, the solvate, the pharmaceutically acceptable salt, the metabolite, or the prodrug thereof according to claim 1, wherein when X’ represents QC(O)R", the compound contains a plurality of basic units $R(X)_n$, wherein one of two adjacent basic units $R(X)_n$ is linked to a skeleton atom of R in another one of the two adjacent basic units $R(X)_n$ through QC(O) in X’, and a recursive hierarchy of the plurality of basic units $R(X)_n$ in the compound comprises not more than 2 levels.

3. The compound, or the stereoisomer, the hydrate, the solvate, the pharmaceutically acceptable salt, the metabolite, or the prodrug thereof according to claim 1, wherein when X’ represents QC(O)R", R" represents the monosaccharide group, and the compound contains a plurality of basic units $R(X)_n$, wherein one of two adjacent basic units $R(X)_n$ is linked to a skeleton atom of R in another one of the two adjacent basic units $R(X)_n$ through the monosaccharide group in X’, and a recursive hierarchy of the plurality of basic units $R(X)_n$ in the compound comprises not more than 2 levels.

4. The compound, or the stereoisomer, the hydrate, the solvate, the pharmaceutically acceptable salt, the metabolite, or the prodrug thereof according to any one of claims 1 to 3, wherein

R is the monosaccharide or the derivative thereof or the aromatic ring, the monosaccharide or the derivative thereof or the aromatic ring being optionally substituted with at least one substituent selected from the group consisting of amino and carboxyl;
R’ represents the optionally substituted six-membered aromatic ring, coumarin, flavone, or isoflavone; and
Q represents -O- or -N-.

5. The compound, or the stereoisomer, the hydrate, the solvate, the pharmaceutically acceptable salt, the metabolite, or the prodrug thereof according to claim 4, wherein the monosaccharide or the derivative thereof is selected from glucose, gluconic acid, or glucosaminic acid, and the aromatic ring is selected from $C_4$-$C_{20}$ aromatic rings.

6. The compound, or the stereoisomer, the hydrate, the solvate, the pharmaceutically acceptable salt, the metabolite, or the prodrug thereof according to claim 5, wherein the aromatic ring is selected from phenyl, chromone, or isoflavone.

7. The compound, or the stereoisomer, the hydrate, the solvate, the pharmaceutically acceptable salt, the metabolite, or the prodrug thereof according to claim 6, wherein

R’ represents the optionally substituted six-membered aromatic ring, coumarin, or flavone, and
Q represents -O-.

8. The compound, or the stereoisomer, the hydrate, the solvate, the pharmaceutically acceptable salt, the metabolite, or the prodrug thereof according to claim 1, wherein each of the at least one basic unit in the compound is one selected from the following structures:

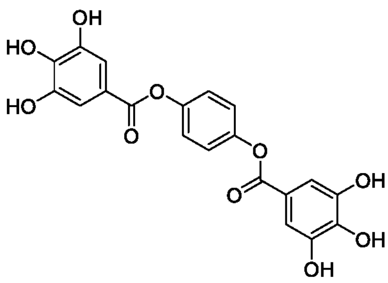

Compound 1

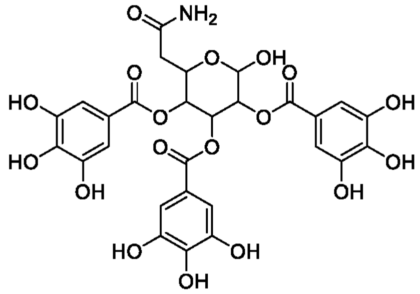

Compound 2

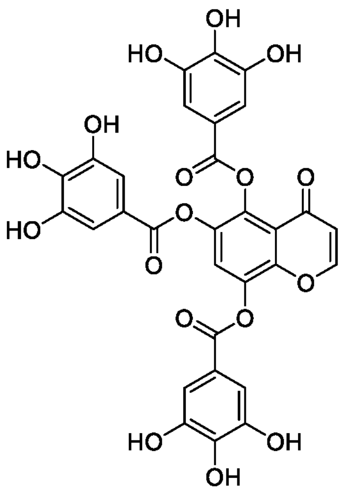

Compound 3

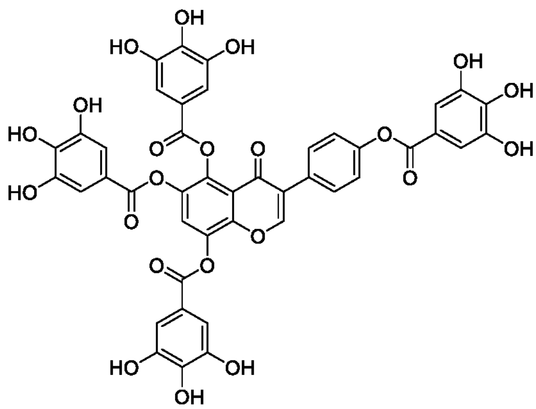

Compound 4

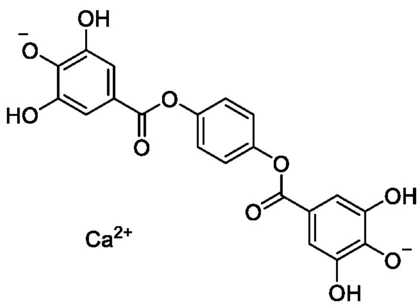

Compound 5

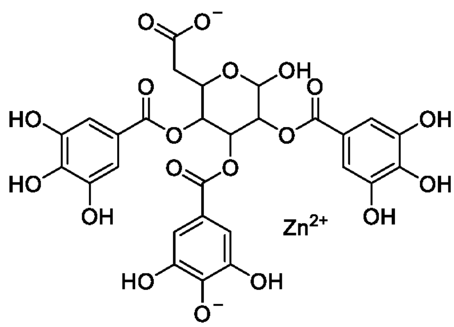

Compound 6

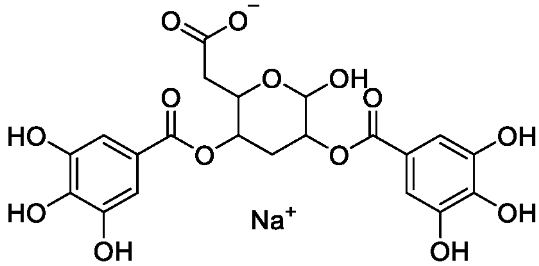

Compound 7.

9. A pharmaceutical composition, comprising the compound, or the stereoisomer, the hydrate, the solvate, the pharmaceutically acceptable salt, the metabolite, or the prodrug thereof according to any one of claims 1 to 8.

10. The pharmaceutical composition according to claim 9, wherein the pharmaceutical composition is in a dosage form selected from powders, tablets, granules, capsules, solutions, emulsions, suspensions, injections, sprays, aerosols, or dry powder inhalations, preferably nasal sprays.

11. Use of the compound, or the stereoisomer, the hydrate, the solvate, the pharmaceutically acceptable salt, the metabolite, or the prodrug thereof according to any one of claims 1 to 8 in the manufacture of drugs for at least one of:

treating or preventing diseases caused by infections of viruses in host cells, preferably the viruses having spike proteins on surfaces thereof;
inhibiting increase of vitamin K; and
inhibiting pulmonary thrombosis.

12. The use according to claim 11, wherein the viruses comprise coronaviruses and influenza viruses.

13. The use according to claim 12, wherein the coronaviruses comprise SARS, MERS, and SARS-CoV-2.

14. The use according to claim 11, wherein the diseases comprise COVID-19, influenza A, and influenza B.

15. Use of a drug in the prevention and/or treatment of diseases caused by infections of viruses in host cells, wherein the drug comprises the compound, or the stereoisomer, the hydrate, the solvate, the pharmaceutically acceptable salt, the metabolite, or the prodrug thereof according to any one of claims 1 to 8 and/or the pharmaceutical composition according to claim 9 or 10, wherein the viruses have spike proteins on surfaces thereof, and the diseases comprise COVID-19, influenza A, and influenza B;

optionally, the viruses comprise coronaviruses and influenza viruses;
optionally, the coronaviruses comprise SARS, MERS, and SARS-CoV-2.

16. Use of a drug in the reduction of a vitamin K level in a patient with increased vitamin K, wherein the drug comprises the compound, or the stereoisomer, the hydrate, the solvate, the pharmaceutically acceptable salt, the metabolite, or the prodrug thereof according to any one of claims 1 to 8 and/or the pharmaceutical composition according to claim 9 or 10.

17. Use of a drug in the prevention and/or treatment of pulmonary thrombosis, wherein the drug comprises the compound, or the stereoisomer, the hydrate, the solvate, the pharmaceutically acceptable salt, the metabolite, or the prodrug thereof according to any one of claims 1 to 8 and/or the pharmaceutical composition according to claim 9 or 10.

18. A method for preventing and/or treating diseases caused by infections of viruses in host cells, wherein the viruses have spike proteins on surfaces thereof, the method comprising:
administering the pharmaceutical composition according to claim 9 or 10 to a patient suffering from or suspected of suffering from the diseases caused by the infections of the viruses in the host cells, wherein:

optionally, the diseases comprise COVID-19, influenza A, and influenza B;
optionally, the viruses comprise coronaviruses and influenza viruses;
optionally, the coronaviruses comprise SARS, MERS, and SARS-CoV-2.

19. A method for reducing a vitamin K level, comprising administering the pharmaceutical composition according to claim 9 or 10 to a patient in need of reducing the vitamin K level.

20. A method for preventing and/or treating pulmonary thrombosis, comprising administering the pharmaceutical composition according to claim 9 or 10 to a patient suffering from or suspected of suffering from the pulmonary thrombosis.

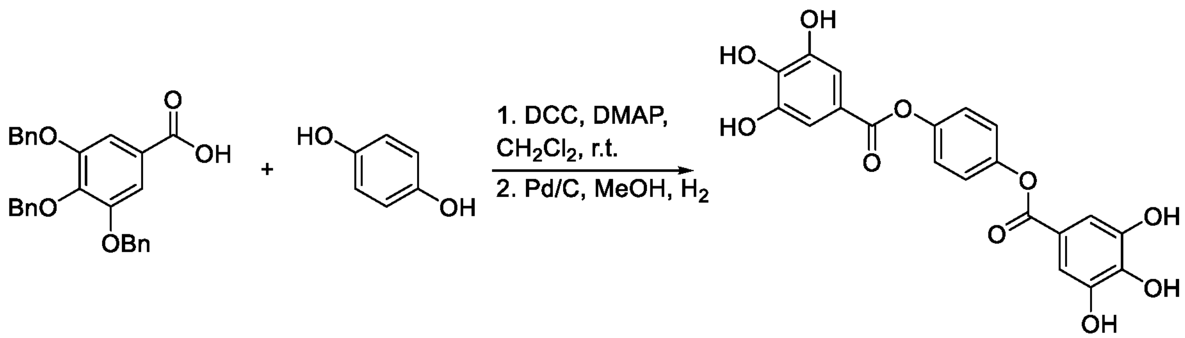


FIG. 1

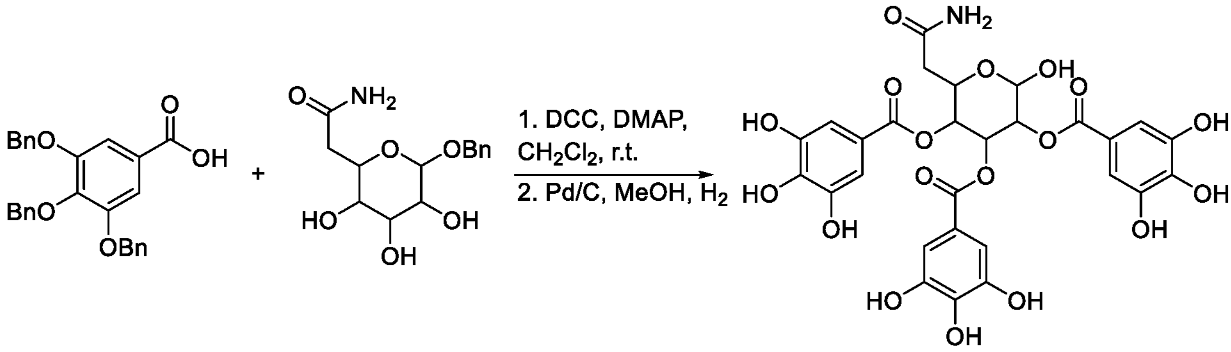


FIG. 2

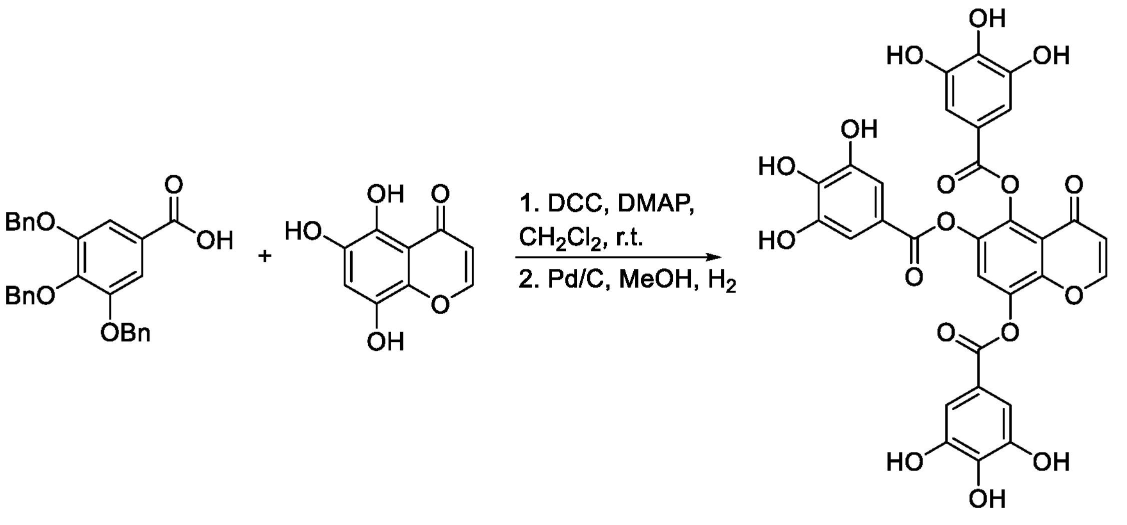


FIG. 3

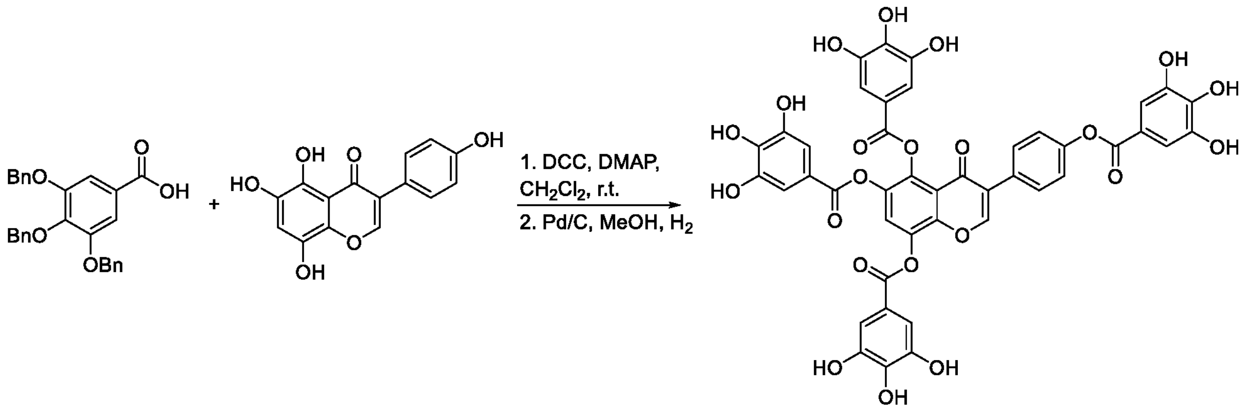


FIG. 4

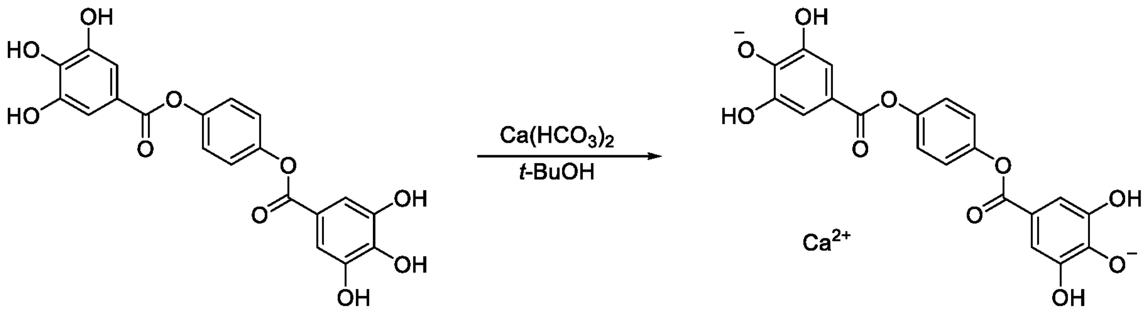


FIG. 5

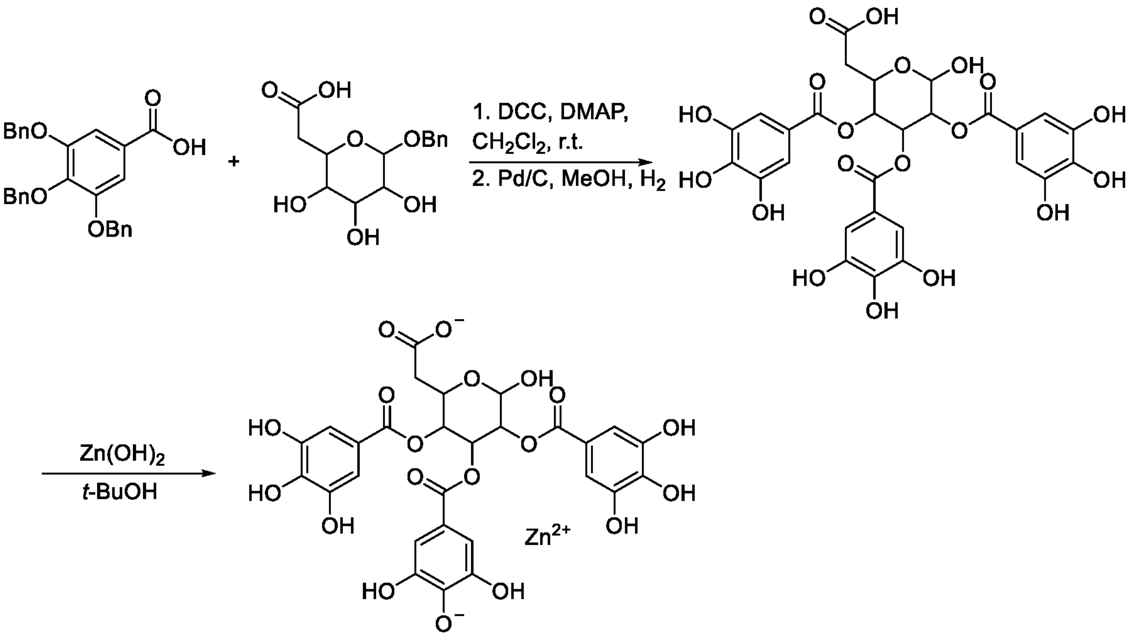


FIG. 6

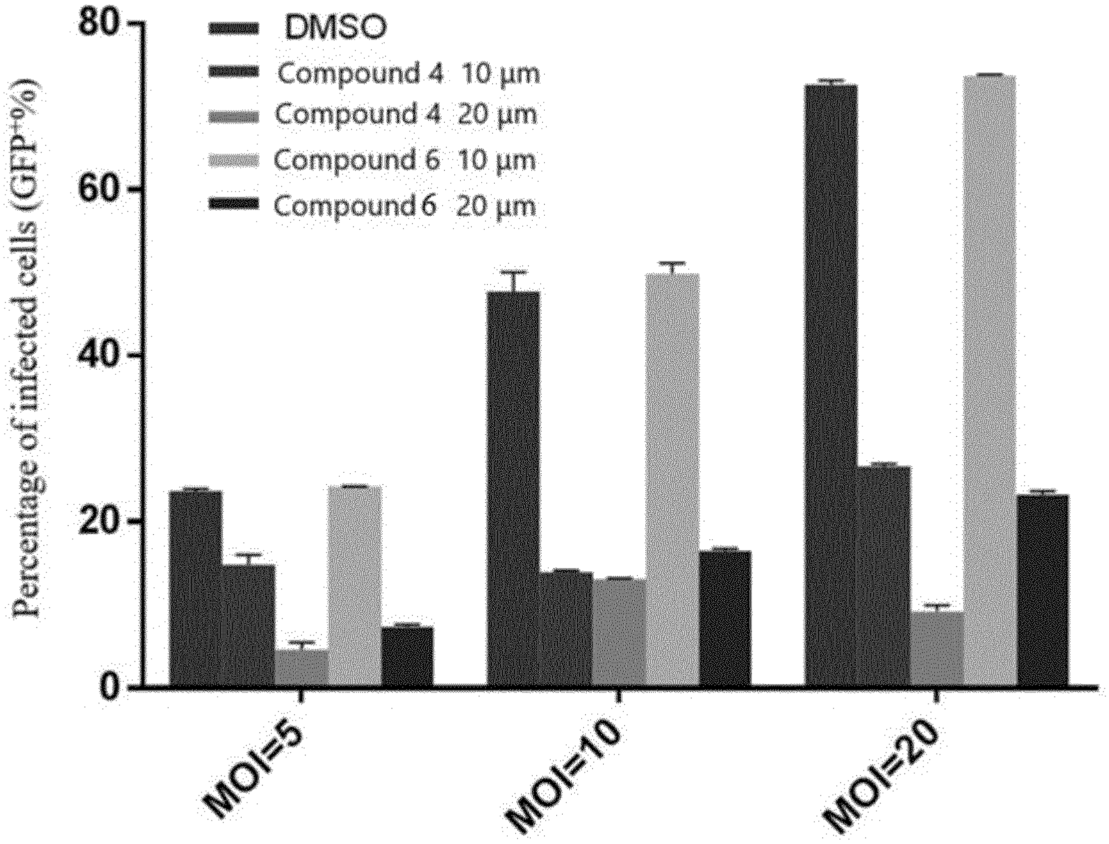
80
60
40
20
0
Percentage of infected cells (GFP+%)
DMSO
Compound 4 10 µm
Compound 4 20 µm
Compound 6 10 µm
Compound6 20 µm
MOI=5
MOI=10
MOI=20

FIG. 7

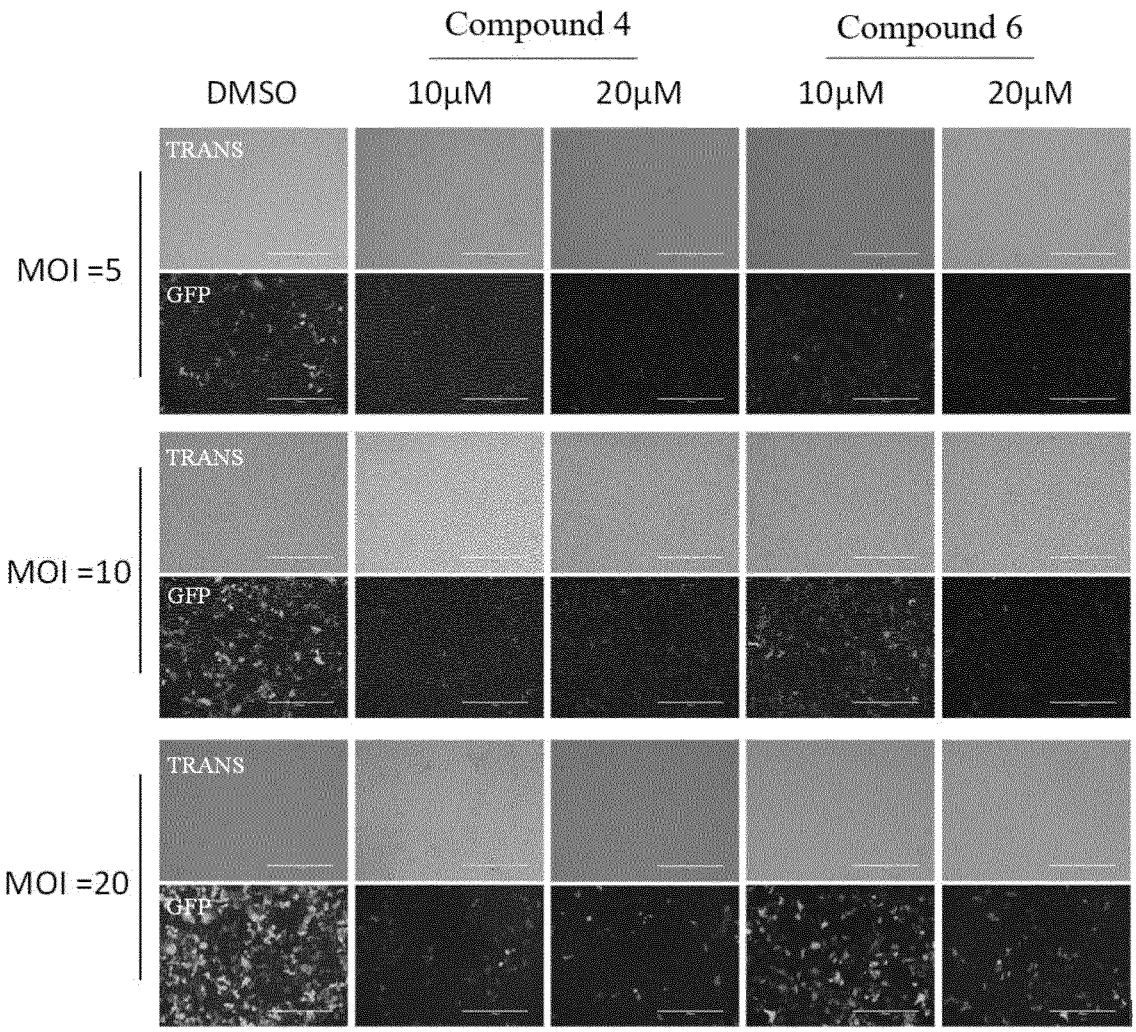
Compound 4
Compound 6
DMSO
10μM
20μM
10μM
20μM
MOI =5
MOI =10
MOI =20
TRANS
GFP
TRANS
GFP
TRANS
GFP

FIG. 8

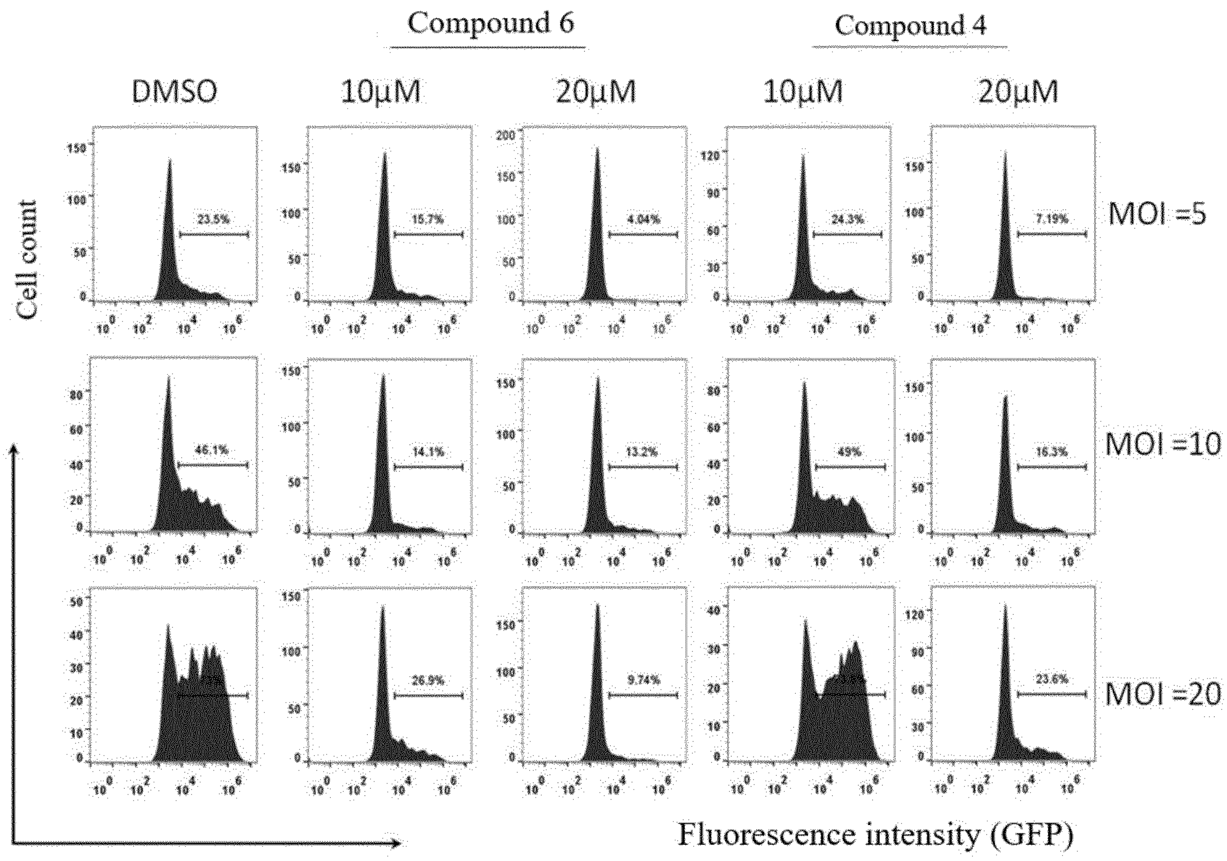
Compound 6
Compound 4
DMSO
10μM
20μM
10μM
20μM
MOI =5
MOI =10
MOI =20
Cell count
Fluorescence intensity (GFP)
23.5%
15.7%
4.04%
24.3%
7.19%
46.1%
14.1%
13.2%
49%
16.3%
26.9%
9.74%
23.6%

FIG. 9

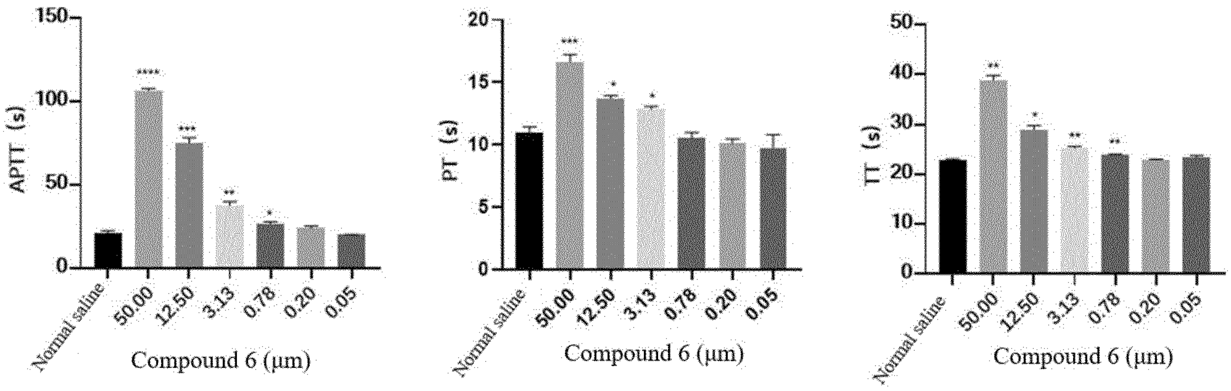
APTT (s)
PT (s)
TT (s)
Normal saline
50.00
12.50
3.13
0.78
0.20
0.05
Compound 6 (μm)

FIG. 10

**INTERNATIONAL SEARCH REPORT**

International application No. **PCT/CN2021/133118**

**A. CLASSIFICATION OF SUBJECT MATTER**

C07H 13/08(2006.01)i; C07D 311/64(2006.01)i; C07C 69/88(2006.01)i; A61K 31/7028(2006.01)i; A61K 31/352(2006.01)i; A61K 31/235(2006.01)i; A61P 31/14(2006.01)i; A61P 9/00(2006.01)i; A61P 11/00(2006.01)i; A61P 35/00(2006.01)i; A61P 19/02(2006.01)i; A61P 29/00(2006.01)i; A61P 13/00(2006.01)i; A61P 1/12(2006.01)i

According to International Patent Classification (IPC) or to both national classification and IPC

**B. FIELDS SEARCHED**

Minimum documentation searched (classification system followed by classification symbols)

C07H; C07D; C07C; A61K; A61P

Documentation searched other than minimum documentation to the extent that such documents are included in the fields searched

Electronic data base consulted during the international search (name of data base and, where practicable, search terms used)

CNABS; DWPI; SIPOABS; CJFD; CNKI; CNTXT; USTXT; WOTXT; EPTXT; STN-REGISTRY; STN-CAPLUS; PATENTICS; 超星读秀; CHAOXING DUXIU; ISI-Web of Science: 深圳市三启药物开发有限公司, 徐峻, 郝梦娇, 张毓婷, 陈浩, 没食子酸, 糖, 异黄酮, 棘蛋白, 病毒, 维生素K, 血栓, 凝血, gallic acid, glucose, ribose, isoflavone, +virus, vitamin K, thrombus, coagulat+, acanthin

**C. DOCUMENTS CONSIDERED TO BE RELEVANT**

| Category* | Citation of document, with indication, where appropriate, of the relevant passages | Relevant to claim No. |
|---|---|---|
| PX | CN 112521432 A (SHENZHEN SANQI DRUG DEVELOPMENT CO., LTD.) 19 March 2021 (2021-03-19)<br>claims 1-14 | 1-20 |
| A | CN 111658631 A (GUANGDONG SUNTRAP LIFE TECHNOLOGY CO., LTD.) 15 September 2020 (2020-09-15)<br>description, paragraphs [0005]-[0028] | 1-20 |
| A | THAPA, M. et al. "Synthesis and Antiviral Activity of Substituted Quercetins"<br>*Bioorganic & Medicinal Chemistry Letters,* Vol. 22, No. 1, 06 November 2011 (2011-11-06), ISSN: 0960-894X,<br>abstract, p. 354, figure 1, p. 356, table 1, and p. 356, column 1, and first paragraph | 1-20 |
| A | TURRODO, C. et al. "New Synthetic Inhibitors of Fatty Acid Synthase with Anticancer Activity"<br>*Journal of Medicinal Chemistry,* Vol. 55, No. 11, 04 May 2012 (2012-05-04), ISSN: 0022-2623,<br>pp. 5013-5023 | 1-20 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **20 January 2022** | **09 February 2022** |

Name and mailing address of the ISA/CN

**China National Intellectual Property Administration (ISA/CN)**
**No. 6, Xitucheng Road, Jimenqiao, Haidian District, Beijing 100088, China**

Facsimile No. **(86-10)62019451**

Authorized officer

Telephone No.

INTERNATIONAL SEARCH REPORT

International application No.
PCT/CN2021/133118

C. DOCUMENTS CONSIDERED TO BE RELEVANT

| Category* | Citation of document, with indication, where appropriate, of the relevant passages | Relevant to claim No. |
|---|---|---|
| A | DESIDERI, N. et al. "Synthesis and Anti-human Immunodeficiency Virus Type 1 Integrase Activity of Hydroxybenzoic and Hydroxycinnamic Acid Flavon-3-yl Esters" *Antiviral Chemistry & Chemotherapy,* Vol. 9, No. 6, 01 December 1998 (1998-12-01), ISSN: 0956-3202, pp. 497-509 | 1-20 |
| A | CONTI, C. et al. "Anti-picornavirus Activity of Synthetic Flavon-3-yl Esters" *Antiviral Chemistry & Chemotherapy,* Vol. 9, No. 6, 01 December 1998 (1998-12-01), ISSN: 0956-3202, pp. 511-515 | 1-20 |

Form PCT/ISA/210 (second sheet) (January 2015)

**INTERNATIONAL SEARCH REPORT**

International application No.
**PCT/CN2021/133118**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **15-20**
because they relate to subject matter not required to be searched by this Authority, namely:

[1] Claim 15 sets forth a use of a medication in the prevention and/or treatment of diseases caused by viral infections of host cells; claim 16 sets forth a use of a medication in lowering a vitamin K level in patients with high vitamin K levels; claim 17 sets forth a use of a medication in the prevention and/or treatment of pulmonary vascular embolism diseases; claim 18 sets forth a method for preventing and/or treating diseases caused by viral infections of host cells; claim 19 sets forth a method for lowering a vitamin K level; and claim 20 sets forth a method for preventing and/or treating pulmonary vascular embolism diseases. The described uses and methods fall within the scope of a method for treatment of a human body, i.e., they fall within the case in which no international search is required as defined in PCT Rule 39.1(iv).

[2] The search on claims 15-20 is made on the basis of the reasonable expectation that said claims are amended as follows: the uses or methods of claims 15-20 are amended to a corresponding use in the preparation of medications.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2021/133118**

| Patent document cited in search report | | | Publication date (day/month/year) | Patent family member(s) | Publication date (day/month/year) |
|---|---|---|---|---|---|
| CN | 112521432 | A | 19 March 2021 | None | |
| CN | 111658631 | A | 15 September 2020 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202011341469


### Non-patent literature cited in the description


- **S.M.BERGE et al.** *J.Pharm.Sci.,* 1977, vol. 66, 1-19